# EUROPEAN PATENT APPLICATION

(11) **EP 3 087 926 A1**
(43) Date of publication of application: **02.11.2016**
(21) Application number: 14874489.9
(22) Date of filing: 26.06.2014
(51) Int. Cl.: A61B 8/14, G01N 29/24

(54) **ULTRASOUND OR PHOTOACOUSTIC PROBE, ULTRASOUND DIAGNOSIS SYSTEM USING SAME, ULTRASOUND THERAPY SYSTEM, ULTRASOUND DIAGNOSIS AND THERAPY SYSTEM, AND ULTRASOUND OR PHOTOACOUSTIC SYSTEM**

(30) Priority: 26.12.2013 KR 20130163518
(71) Applicant: Nohsn Co., Ltd., Iksan-si, Jeollabuk-do 570-802 (KR)
(72) Inventor: HAN, Cheol Min, Jeonju-si Jeollabuk-do 561-802 (KR)
(74) Representative: Zinnecker, Armin
(86) International application number: PCT/KR2014/005681
(87) International publication number: WO 2015/099253

(57) **Abstract**

Provided are an ultrasound or photoacoustic probe, an ultrasound diagnosis system using the same, an ultrasound therapy system, an ultrasound diagnosis and therapy system, and an ultrasound or photoacoustic system. The ultrasound or photoacoustic probe, according to one embodiment of the present invention, includes: a first transmitting and receiving unit transmitting an ultrasound or photoacoustic signal to an object; and a second transmitting and receiving unit disposed to face the first transmitting and receiving unit, and receiving the ultrasound or photoacoustic signal passed through the object.

## Description

### [Technical Field]

The present invention is relates to an ultrasound or photoacoustic probe, an ultrasound diagnosis system using the same, an ultrasound therapy system, an ultrasound diagnosis and therapy system, and an ultrasound or photoacoustic system.

### [Background Art]

Generally, an ultrasound system using an industrial or medical ultrasound apparatus uses a scheme of transmitting ultrasound to an object and receiving reflected echo-signals to acquire images of the object. That is, an ultrasound probe according to the related art was configured to perform transmission and reception of ultrasound in a single element. However, it is difficult to receive echo-signals in an object having a high hardness such as the bones, the teeth, or the like, due to physical properties of the ultrasound, and thus, it is difficult to acquire the image of the object for diagnosis and treatment. Particularly, in the case of a dental diagnostic image apparatus, due to the difficulty as described above, image information of the object is generally acquired using a dental computed tomography (CT) or X-ray apparatus. However, a dental CT or X-ray apparatus has a disadvantage such as exposure to radiation, and it is difficult to acquire diagnostic image in real time to apply dental operation.

### [Related Art Document]

### [Patent Document]

Korean Patent Publication No. 10-1243499 (March 13, 2013)

### [Disclosure]

### [Technical Problem]

An object of the present invention is to provide an ultrasound or photoacoustic probe for acquiring an image of an object having a high hardness in real time without a risk of exposure to radiation, and a system using the same.

### [Technical Solution]

In one general aspect, an ultrasound or photoacoustic probe includes: a first transmitting and receiving unit for transmitting an ultrasound or photoacoustic signal to an object; and a second transmitting and receiving unit disposed to face the first transmitting and receiving unit, and receiving the ultrasound or photoacoustic signal passed through the object.

The ultrasound or photoacoustic probe may further include a distance adjusting unit adjusting a distance between the first and second transmitting and receiving units.

The ultrasound or photoacoustic probe may have at least one shape of a ' 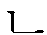 ' shape, a ' 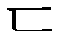 ' shape, a '11' shape, and a '=' shape.

Each of the first and second transmitting and receiving units may include a plurality of elements generating the ultrasound or photoacoustic signal.

The elements generating the ultrasound or photoacoustic signal may be elements for diagnosis or therapy.

The ultrasound or photoacoustic probe may further include a wired or wireless transmitting and receiving unit.

The ultrasound or photoacoustic probe may further include a gel pad suitable for properties of a medium.

The gel pad may be formed in a shape corresponding to the ultrasound or photoacoustic probe.

In another general aspect, an ultrasound diagnosis system using the ultrasound or photoacoustic probe as described above includes: a diagnosis function unit; a signal generating unit generating a diagnosis pulse for diagnosis of the object when the diagnosis function unit is activated; and a diagnostic image generating unit generating a diagnostic image for diagnosis of the object using the diagnosis pulse.

In another general aspect, an ultrasound therapy system using the ultrasound or photoacoustic probe as described above includes: a treatment function unit; and a signal generating unit generating a treatment pulse for treatment of the object when the treatment function unit is activated.

In another general aspect, an ultrasound diagnosis and therapy system using the ultrasound or photoacoustic probe as described above includes: a diagnosis function unit; a treatment function unit; a signal generating unit generating a diagnosis pulse for diagnosis of the object when the diagnosis function unit is activated, and generating a treatment pulse for treatment of the object when the treatment function unit is activated; a diagnostic image generating unit generating a diagnostic image for diagnosis of the object using the diagnosis pulse; a therapeutic virtual image generating unit generating a therapeutic virtual image for treatment of the object using the treatment pulse; and a therapeutic image generating unit generating a therapeutic image using the diagnostic image and the therapeutic virtual image.

In another general aspect, an ultrasound or photoacoustic system using the ultrasound or photoacoustic probe as described above includes: a therapeutic virtual image generating unit generating virtual images of a therapeutic region, or treatment beam, and a therapeutic beam field, or treatment beam field, of a transmission signal for treatment.

The ultrasound or photoacoustic system may further include an operation information managing unit including information on at least one of an operation site, an operation range, and an operation method.

The ultrasound or photoacoustic system may further include a safety controlling unit securing safety of an operation according to the information of the operation information managing unit.

The ultrasound or photoacoustic system may further include: a diagnosis function unit; a treatment function unit; and a signal generating unit generating a diagnosis pulse for diagnosis of the object when the diagnosis function unit is activated, and generating a treatment pulse for treatment of the object when the treatment function unit is activated, wherein a signal generated in the signal generating unit has a delayed signal value so that any one of the first and second transmitting and receiving units has directivity and generates a steering signal, and a 2D/3D/4D image is formed by analyzing the received signal according to the delayed signal value.

The signal generated in the signal generating unit may be controlled so as to be steered to one surface disposed to face any one of the first and second transmitting and receiving units in any one direction of upward, downward, left, and right directions.

### [Advantageous Effects]

According to exemplary embodiments of the present invention, since the first transmitting and receiving unit 102 of the ultrasound or photoacoustic probe transmits the ultrasound to the object, and the second transmitting and receiving unit 104 disposed to face the first transmitting and receiving unit 102 receives the ultrasound passed through the object, the images of the object having a high hardness such as the jawbone, the teeth, or the like, may be obtained in real time, thereby making it possible to easily perform diagnosis, treatment, or the like, of the teeth, the jawbone, or the like.

### [Description of Drawings]

FIG. 1 is a diagram for explaining a detailed configuration of an ultrasound or photoacoustic probe according to a first exemplary embodiment of the present invention.
FIG. 2 is a diagram illustrating a shape of the ultrasound or photoacoustic probe according to the first exemplary embodiment of the present invention to which a gel pad is attached and which is covered by a protection cover.
FIG. 3 is a diagram for explaining a detailed configuration of an ultrasound or photoacoustic probe according to a second exemplary embodiment of the present invention.
FIG. 4 is a diagram illustrating a shape of the ultrasound or photoacoustic probe according to the second exemplary embodiment of the present invention to which a gel pad is attached and on which a protection cover is mounted.
FIG. 5 is a diagram illustrating a state in which an ultrasound system according to an exemplary embodiment of the present invention is connected to the ultrasound or photoacoustic probe.
FIG. 6 is a block diagram illustrating a detailed configuration of the ultrasound system according to the exemplary embodiment of the present invention.
FIG. 7 is a block diagram illustrating a detailed configuration of a setting unit according to an exemplary embodiment of the present invention.
FIG. 8 is a diagram illustrating an arrangement configuration of ultrasound vibration devices according to the exemplary embodiment of the present invention.
FIG. 9 is a block diagram illustrating the ultrasound or photoacoustic probe according to the exemplary embodiment of the present invention, the ultrasound system, and an external output apparatus.
FIG. 10 is a block diagram illustrating an image generating unit according to an exemplary embodiment of the present invention.
FIG. 11 is a block diagram including an operation information managing unit and a safety controlling unit according to an exemplary embodiment of the present invention.
FIG. 12 is a block diagram illustrating a therapeutic beam field, or treatment beam field, and a therapeutic region, or treatment beam, which are virtual images for treatment of a convex ultrasound or photoacoustic probe according to an exemplary embodiment of the present invention.
FIG. 13 is a treatment image in which the therapeutic beam field, or treatment beam field, and the therapeutic region, or treatment beam of the probe, which are virtual images for treatment, are synthesized with a 2D diagnostic image according to an exemplary embodiment of the present invention.
FIG. 14 is an image illustrating a method of generating a pulse to the object in any one of first and second transmitting and receiving units 102 and 104 according to an exemplary embodiment of the present invention.

### [Best Mode]

The present invention may be variously modified and have various types, and specific embodiments of the present invention will be described in detail with reference to the accompanying drawing. However, the present invention is not limited to the exemplary embodiments described herein, but all of the modifications, equivalents, and substitutions within the spirit and scope of the present invention are also included in the present invention.

In describing the present invention, when a detailed description of well-known technology relating to the present invention may unnecessarily make unclear the gist of the present invention, a detailed description thereof will be omitted. Further, the following terminologies are defined in consideration of the functions in the present invention and may be construed in different ways by the intention of users and operators, conventions, or the like. Therefore, the definitions thereof should be construed based on the contents throughout the specification.

As a result, the technical spirit of the present invention is determined by the claims and the following exemplary embodiments may be provided to efficiently describe the spirit of the present invention to those skilled in the art.

Hereinafter, exemplary embodiments of the present invention will be described with reference to the accompanying drawings. However, the exemplary embodiments are described by way of examples only and the present invention is not limited thereto.

According to an exemplary embodiment of the present invention, first and second transmitting and receiving units 102 and 104 are functional units including a single element or a plurality of elements and transmitting an ultrasound or photoacoustic signal through the element or receiving an echoed signal or transmitted signal. The first and second transmitting and receiving units 102 and 104 may be represented by first and second faces 102 and 104, respectively. Here, any one of the first and second transmitting and receiving units 102 and 104 may transmit the ultrasound or photoacoustic signal, and the other may receive the transmitted signal passed through an object.

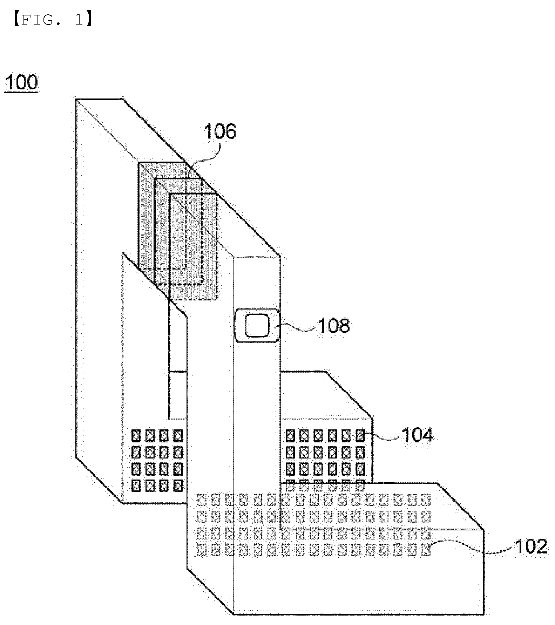

FIG. 1 is a diagram for explaining a detailed configuration of an ultrasound or photoacoustic probe 100 according to a first exemplary embodiment of the present invention. As illustrated in FIG. 1, the ultrasound or photoacoustic probe 100 according to the first exemplary embodiment of the present invention includes a first transmitting and receiving unit 102, a second transmitting and receiving unit 104, a distance adjusting unit 106, an interface unit 108, a gel pad 110, and a protection cover 112.

The first and second transmitting and receiving units 102 and 104, which are arrays of a plurality of elements transmitting and receiving ultrasound, transmit the ultrasound to an object (not illustrated) or receive the ultrasound passed through the object. An array form of the elements may be variously changed depending on the kind or physical properties of object. The first and second transmitting and receiving units 102 and 104 are disposed to face each other, and the object may be disposed between the first and second transmitting and receiving units 102 and 104. Here, the object is a target object for securing diagnostic images using an ultrasound or photoacoustic diagnosis pulse or a target object for treatment or destruction using an ultrasound or photoacoustic treatment pulse. For example, the object may be the teeth, the bone, the jawbone, cement, semiconductors, the skin, and the like. As illustrated in FIG. 1, the ultrasound or photoacoustic probe 100 according to the first exemplary embodiment of the present invention may be formed in a ' 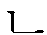 ' or ' 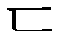 ' shaped symmetrical structure. In this case, the first and second transmitting and receiving units 102 and 104 may be disposed to be spaced apart from each other and to face each other. When the first and second transmitting and receiving units 102 and 104 are disposed to face each other, the ' 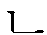 ' or ' 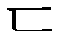 ' shaped symmetrical structure is only an example, and a shape of the symmetrical structure may be various such as a '11' shape and a '=' shape.

Since the first and second transmitting and receiving units 102 and 104 are disposed to face each other, in the case in which the first transmitting and receiving unit 102 transmits the ultrasound to the object, the second transmitting and receiving unit 104 may receive the ultrasound passed through the object. Similarly, in the case in which the second transmitting and receiving unit 104 transmits the ultrasound to the object, the first transmitting and receiving unit 102 may receive the ultrasound passed through the object. The first and second transmitting and receiving units 102 and 104 may each perform a function of any one of an ultrasound transmitter (TX) and an ultrasound receiver (RX) according to a state of the object or a manual operation of a user. As described above, it is difficult to receive an echo-signal in an object having a high hardness such as the teeth, the jawbones, or the like, due to physical properties of the ultrasound, and thus, it is difficult to acquire image of the objects for ultrasound diagnosis or treatment, or the like. Therefore, according to exemplary embodiments of the present invention, since the ultrasound or photoacoustic probe 100 is configured so that the first transmitting and receiving unit 102 transmits the ultrasound to the object, and the second transmitting and receiving unit 104 disposed to face the first transmitting and receiving unit 102 receives the ultrasound passed through the object, images of the object having a high hardness such as the teeth, the jawbone, or the like, may be acquired in real time, thereby making it possible to easily perform diagnosis, treatment, or the like, of the teeth, the jawbone, or the like.

The ultrasound or photoacoustic probe 100 according to the present invention does not receive only the signals transmitted by the first and second transmitting and receiving units 102 and 104 facing each other, and in the case in which the echoed signal may be interpreted depending on a hardness of a medium, diagnostic images may be formed by interpreting the echoed RX signal. This uses the physical properties of the ultrasound or photoacoustic probe, and the present invention is not limited to formation of the diagnostic images using only the signal transmitted from the object.

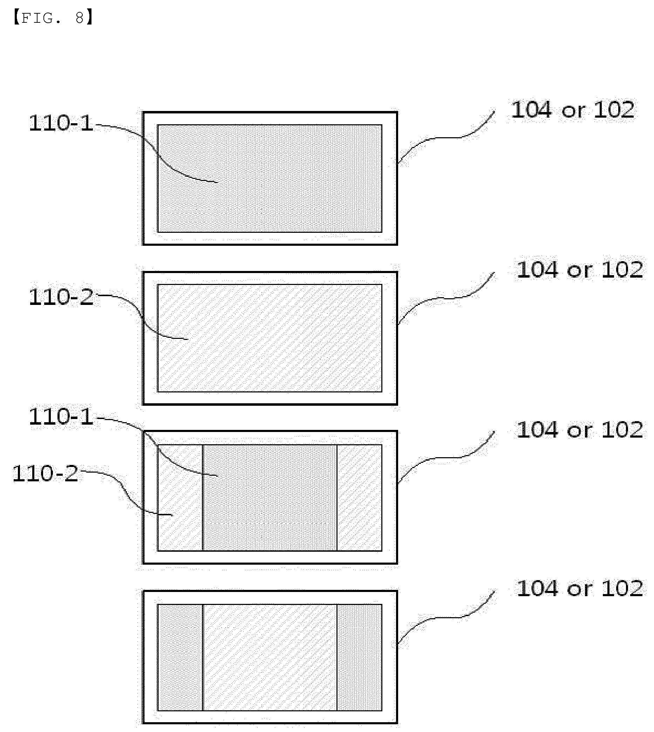

The first and second transmitting and receiving units 102 and 104 may be formed of ultrasound vibration devices 110-1 for diagnosis or ultrasound vibration devices 110-2 for treatment as illustrated in FIG. 8, or formed of a combination of the ultrasound vibration device for diagnosis and the ultrasound vibration device for treatment.

As an example, the first and second transmitting and receiving units 102 and 104 are formed of the ultrasound vibration devices for diagnosis as described above, which is to acquire diagnostic images of the object, and any one of the first and second transmitting and receiving units 102 and 104 is formed of the ultrasound vibration device for treatment, which is to treat the object. Further, in the case in which the first and second transmitting and receiving units 102 and 104 are combined (disposed) as the ultrasound vibration device for diagnosis and the ultrasound vibration device for treatment, the ultrasound or photoacoustic probe may perform a diagnosis function and a treatment function.

FIG. 8 is an example of a configuration (arrangement) of the ultrasound vibration devices of the first and second transmitting and receiving units 102 and 104, and first and second transmitting and receiving units 102 and 104 may be implemented in various shapes depending on diagnostic or therapeutic purposes of the ultrasound vibration device or the ultrasound or photoacoustic probe.

Meanwhile, even though ultrasound having the same frequency is transmitted to the same object at different positions from each other, quality of the images of the object acquired from the ultrasound passed through the object may be changed depending on a structure and a shape of the object. Further, there is a need to transmit ultrasound having different frequencies to the object depending on the kind and properties of the object, and the like, to acquire images of the object in different frequency bands. Therefore, according to exemplary embodiments of the present invention, the ultrasound or photoacoustic probe is configured so that the first transmitting and receiving unit 102 may sequentially serve as the ultrasound transmitter (TX) and the ultrasound receiver (RX) and the second transmitting and receiving unit 104 may sequentially serve as the ultrasound receiver (RX) and the ultrasound transmitter (TX) with respect to the same object, such that higher quality images of the object may be acquired. As described above, the first transmitting and receiving unit 102 may transmit the ultrasound to the object, and the second transmitting and receiving unit 104 may receive the ultrasound passed through the object. Thereafter, the second transmitting and receiving unit 104 may transmit ultrasound having a frequency equal to or different from the frequency of the ultrasound transmitted to the object by the first transmitting and receiving unit 102 to the object, and the first transmitting and receiving unit 102 may receive the ultrasound transmitted from the second transmitting and receiving unit 104 and passed through the object. That is, in the case in which alternate transmission and reception of the ultrasound is required, the first transmitting and receiving unit 102 may sequentially serve as the ultrasound transmitter (TX) and the ultrasound receiver (RX), and the second transmitting and receiving unit 104 may sequentially serve as the ultrasound receiver (RX) and the ultrasound transmitter (TX).

The distance adjusting unit 106 adjusts a distance between the first and second transmitting and receiving units 102 and 104. As described above, the object may be, for example, the teeth, the jawbones, or the like. In this case, the ultrasound or photoacoustic probe may have various structures and shapes depending on the kind of subject or object. Therefore, the distance adjusting unit 106 may adjust the distance between the first and second transmitting and receiving units 102 and 104 depending on the kind or structure of the object. In detail, the distance adjusting unit 106 may adjust the distance between the first and second transmitting and receiving units 102 and 104 using a distance measurement signal received from a sensor (not illustrated) measuring the distance between the first and second transmitting and receiving units 102 and 104. For example, a micro actuator may be mounted in the distance adjusting unit 106, and the sensor may sense the number of rotations of the micro actuator. As the distance between the first and second transmitting and receiving units 102 and 104 is changed, the number of rotations of the micro actuator in the distance adjusting unit 106 may also be changed. For example, as the distance between the first and second transmitting and receiving units 102 and 104 is increased, the number of rotations of the micro actuator may also be increased. The sensor may sense the number of rotations of the micro actuator to generate the distance measurement signal, and transmit the generated distance measurement signal to the distance adjusting unit 106. The distance adjusting unit 106 may receive the distance measurement signal from the sensor to adjust the distance between the first and second transmitting and receiving units 102 and 104. Further, the sensor may sense the number of rotations of the micro actuator to transmit the sensed number of rotations to an ultrasound system 500 as described below. In this case, the distance adjusting unit 106 may receive a distance control signal from the ultrasound system 500 to adjust the distance between the first and second transmitting and receiving units 102 and 104. The sensor may be attached to the distance adjusting unit 106 or suitably disposed at another position of the ultrasound or photoacoustic probe 100. Meanwhile, for example, the distance adjusting unit 106 may connect one side surface of the ultrasound or photoacoustic probe 100 and the other side surface of the ultrasound or photoacoustic probe 100 opposing one side surface thereof to each other at a predetermined thickness, and adjust the distance between the first and second transmitting and receiving units 102 and 104 by adjusting the thickness between one side surface and the other side surface of the ultrasound or photoacoustic probe 100 as illustrated, but is not limited thereto.

As a distance measurement source used in the distance adjusting unit 106, ultrasound, light, or the like, may be used, and as a mechanical method, a method using an actuator as described above may be used. Further, in the case in which the ultrasound or photoacoustic probes 100 and 200 according to the exemplary embodiments of the present invention have a ' 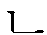 ' or ' 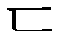 ' shape, the distance adjusting unit 106 may be embedded in the ultrasound or photoacoustic probe 100 according to the present invention, and in the case in which the ultrasound or photoacoustic probes 100 and 200 according to the exemplary embodiments of the present invention have a '11' or '=' shape, since the first face 102 and the second transmitting and receiving unit 104 may face each other such as a rail, an arm, or the like, the distance adjusting unit 106 may be disposed outside.

In addition, the distance adjusting unit 106 may adjust the distance between the first and second transmitting and receiving units 102 and 104 according to a distance control signal received from the outside. As described below, the ultrasound or photoacoustic probe 100 may be connected to the ultrasound system 500 by a wired or wireless connection method. The distance adjusting unit 106 may receive the distance control signal from the ultrasound system 500 to adjust the distance between the first and second transmitting and receiving units 102 and 104 according to the distance control signal. The distance control signal, which is generated based on the distance between the first and second transmitting and receiving units 102 and 104 calculated in the ultrasound system 500, may be received from the ultrasound system 500. The distance control signal may be generated based on a distance calculated using the number of rotations of the micro actuator as described above.

In addition, the distance adjusting unit 106 may adjust the distance between the first and second transmitting and receiving units 102 and 104 according to a manual operation of a user, that is, a physical operation the user. As described above, the distance adjusting unit 106 may connect, for example, one side surface of the ultrasound or photoacoustic probe 100 and the other side surface of the ultrasound or photoacoustic probe 100 opposing one side surface thereof to each other at a predetermined thickness. The user may adjust the thickness between the one side surface and the other side surface of the ultrasound or photoacoustic probe 100 to adjust the distance between the first and second transmitting and receiving units 102 and 104.

The interface unit 108 may serve as an interface between the ultrasound or photoacoustic probe 100 and the ultrasound system 500, and transmit and receive electrical signal with the ultrasound system 500. The interface unit 108 may be, for example, a connector. The interface unit 108 may receive a transmission signal, which is an electrical conversion signal of the ultrasound to be transmitted to the object by the first transmitting and receiving unit 102, from the ultrasound system 500, or may transmit a reception signal, which is an electrical conversion signal of the ultrasound received from the object by the second transmitting and receiving unit 104, to the ultrasound system 500. Further, the interface unit 108 may receive the distance control signal for adjusting the distance between the first and second transmitting and receiving units 102 and 104 from the ultrasound system 500, and may also receive a control signal for controlling each of the configurations of the ultrasound or photoacoustic probe 100.

The gel pad 110, which is to prevent ultrasound attenuation, may be attached to outer surfaces of the first and second transmitting and receiving units 102 and 104. Physical properties of the gel pad 110 may be changed depending on properties of a medium. The reason of using a gel pad 110 having different physical properties depending on the properties of the medium is to match acoustic impedance of the gel pad 110 with the object.

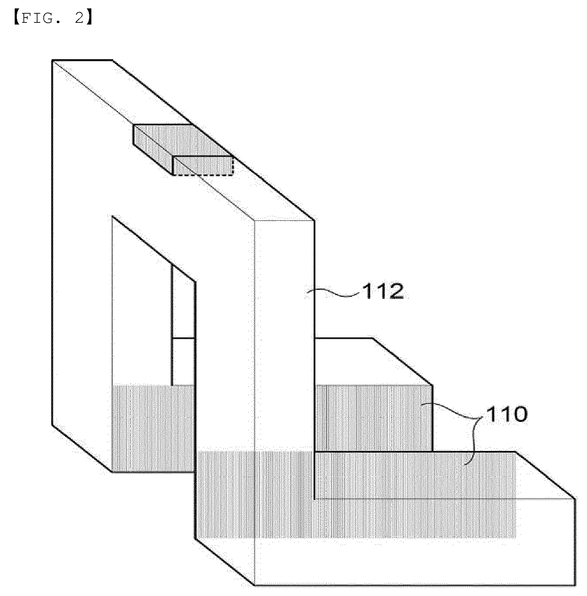

The protection cover 112, which is to protect the ultrasound or photoacoustic probe 100 from external impact and prevent contamination by a contaminated material (for example, saliva), may be formed to enclose an outer surface of the ultrasound or photoacoustic probe 100. The gel pad 110 and the protection cover 112 will be described in detail with reference to FIG. 2.

FIG. 2 is a diagram illustrating a shape of the ultrasound or photoacoustic probe 100 according to the first exemplary embodiment of the present invention to which the gel pad 110 is attached, and which is covered with the protection cover 112.

As illustrated in FIG. 2, the gel pad 110 may be attached to the outer surfaces of the first and second transmitting and receiving units 102 and 104. The gel pad 110, which is to prevent ultrasound attenuation, may be a pad formed of a flexible material. The gel pad 110 may be attached to the outer surface of each of the first and second transmitting and receiving units 102 and 104 to contact the object (for example, the teeth), and may be deformed depending on the structure and the shape of the object. That is, the gel pad 110 may be deformed to a discontinuous curved surface depending on the structure and the shape of the object to thereby be closely adhered to the object. As the gel pad 110 is deformed so as to be closely adhered to the object, a contact area between the gel pad 110 and the object may be increased, and thus, ultrasound attenuation prevent efficiency of the gel pad 110 may be further increased.

Next, the protection cover 112 may be formed to enclose the outer surface of the ultrasound or photoacoustic probe 100. The protection cover 112 encloses the outer surface of the ultrasound or photoacoustic probe 100, thereby making it possible to protect the ultrasound or photoacoustic probe 100 from external impact and prevent contamination by the contaminated material (for example, saliva). In addition, the protection cover 112 may have an effect of preventing ultrasound attenuation, similarly to the gel pad 110, and may perform a waterproof function of the ultrasound or photoacoustic probe 100. The protection cover 112 may be separated into a predetermined number of regions, and the separated regions may be coupled to each other so as to enclose the outer surface of the ultrasound or photoacoustic probe 100. The protection cover 112 may be configured so that the protection cover 112 may be separated and detached from the ultrasound or photoacoustic probe 100 at any time, and whenever the object is changed, the protection cover may be replaced. That is, the protection cover 112 may be configured so as to be used only once and be replaced every time. The protection cover 112 may be made of the same material as that of the gel pad 110, but is not limited thereto.

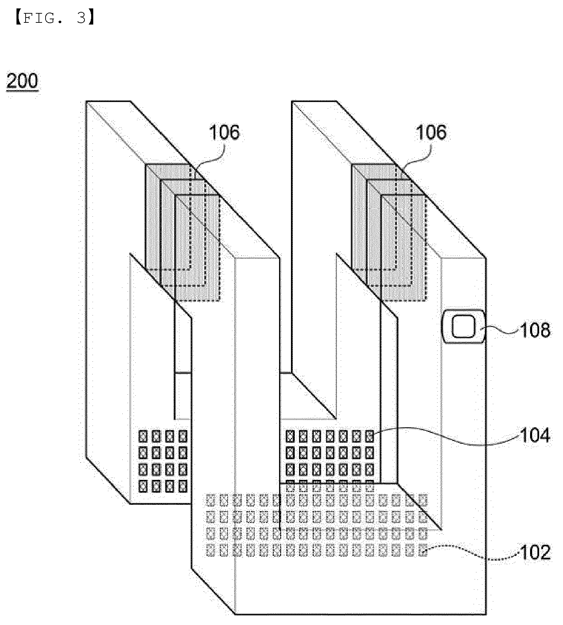

FIG. 3 is a diagram for explaining a detailed configuration of an ultrasound or photoacoustic probe 200 according to a second exemplary embodiment of the present invention. As illustrated in FIG. 3, the ultrasound or photoacoustic probe 200 according to the second exemplary embodiment of the present invention may be formed in a ' 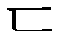 ' shaped symmetrical structure. Since detailed configurations and functions of the ultrasound or photoacoustic probe 200 according to the second exemplary embodiment of the present invention are the same as described above, a detailed description thereof will be omitted, and reference numerals used above will be equally used. Meanwhile, as illustrated in FIG. 3, the ultrasound or photoacoustic probe 200 according to the second exemplary embodiment of the present invention may include a plurality of distance adjusting units 106. In this case, the plurality of distance adjusting units 106 may be disposed to face each other, and may operate together with each other.

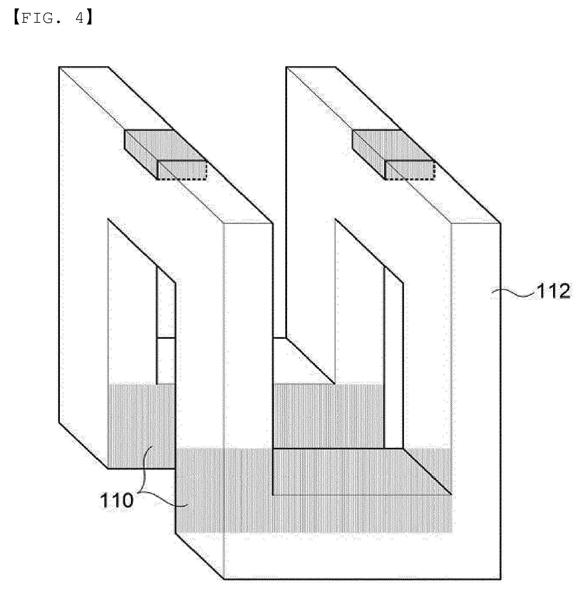

FIG. 4 is a diagram illustrating a shape of the ultrasound or photoacoustic probe 200 according to the second exemplary embodiment of the present invention to which a gel pad 110 is attached and on which a protection cover 112 is mounted. As described above, the gel pad 110, which is to prevent ultrasound attenuation, may be attached to outer surfaces of first and second transmitting and receiving units 102 and 104. The protection cover 112, which is to protect the ultrasound or photoacoustic probe 200 from external impact and prevent contamination by a contaminated material, may be formed to enclose outer surfaces of the ultrasound or photoacoustic probe 200. As illustrated in FIG. 4, a shape of the protection cover 110 may be changed depending on a shape of the ultrasound or photoacoustic probe 100 or 200, but the functions thereof are the same as described above.

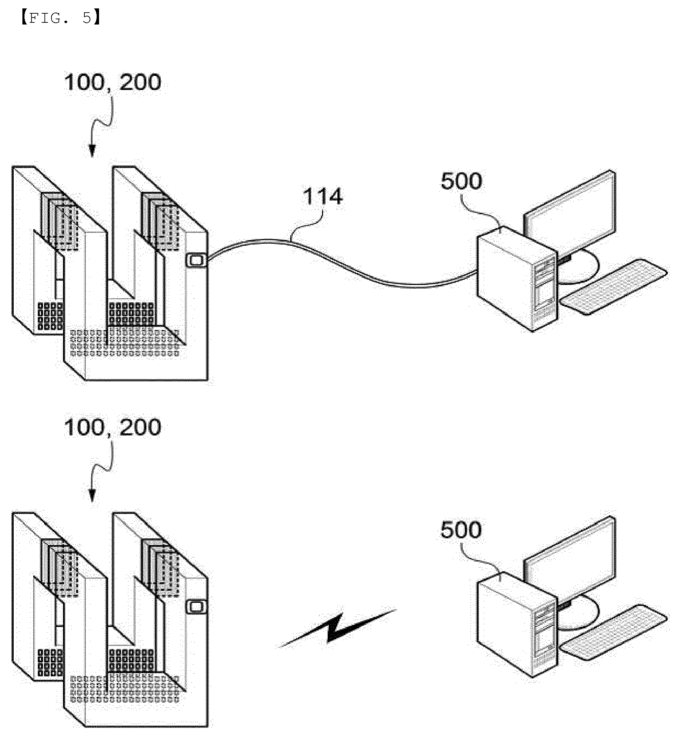

FIG. 5 is a diagram illustrating a state in which an ultrasound system 500 according to an exemplary embodiment of the present invention is connected to the ultrasound or photoacoustic probe 100 or 200. As described above, the ultrasound or photoacoustic probe 100 or 200 according to the exemplary embodiment of the present invention may be connected to the ultrasound system 500 by the wired or wireless connection method, and may transmit and receive electrical signals with the ultrasound system 500. In the case in which the ultrasound or photoacoustic probe 100 or 200 is connected to the ultrasound system 500 by the wired connection method, the interface unit 108 of the ultrasound or photoacoustic probe 100 or 200 may be connected to the ultrasound system 500 through a cable 114.

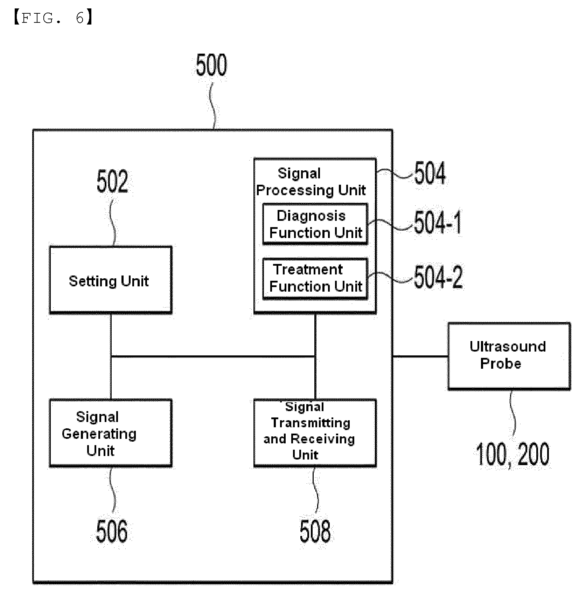

FIG. 6 is a block diagram illustrating a detailed configuration of the ultrasound system 500 according to the exemplary embodiment of the present invention. As illustrated in FIG. 6, the ultrasound system 500 according to the exemplary embodiment of the present invention may include a setting unit 502, a signal processing unit 504, a signal generating unit 506, and a signal transmitting and receiving unit 508, and be connected to the ultrasound or photoacoustic probe 100 or 200 by the wired or wireless connection method.

The setting unit 502 activates the signal processing unit 504 of the ultrasound system 500 before the ultrasound or photoacoustic probe 100 or 200 transmits ultrasound to the object, or performs a pre-processing process for setting functions and positions of the first and second transmitting and receiving units 102 and 104 of the ultrasound or photoacoustic probe 100 or 200. The setting unit is a functional unit setting set values required in order to secure diagnostic images of the object such as a frequency and an amplitude of an ultrasound pulse, power intensity, a channel group, the number of channels, steering, a depth, distance information, or the like, and setting values required in order to transmit treatment pulse to the object.

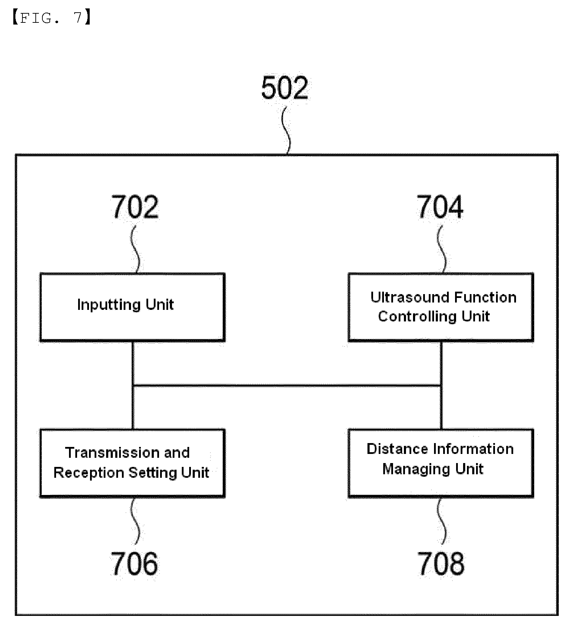

FIG. 7 is a block diagram illustrating a detailed configuration of the setting unit 502 according to the exemplary embodiment of the present invention. As illustrated in FIG. 7, the setting unit 502 includes an inputting unit 702, an ultrasound function controlling unit 704, a transmission and reception setting unit 706, and a distance information managing unit 708.

The inputting unit 702 receives an input signal from the outside. Here, the input signal may be, for example, diagnosis signal information for diagnosis of the object or treatment signal information for treatment of the object, and be inputted by the user. A diagnosis signal and a treatment signal may have, for example, different frequencies from each other.

The ultrasound function controlling unit 704 activates any one of a diagnosis function unit 504-1 and a treatment function unit 504-2 of the signal processing unit 504 according to the input signal. In the case in which the diagnosis function unit 504-1 is activated according to the input signal inputted in the inputting unit 702, the signal generating unit 506 generates a transmission signal for diagnosis of the object. Further, in the case in which the treatment function unit 504-2 is activated according to the input signal inputted in the inputting unit 702, the signal generating unit 506 generates a transmission signal for treatment of the object.

The transmission and reception setting unit 706 sets each of the first and second transmitting and receiving units 102 and 104 of the ultrasound or photoacoustic probe 100 or 200 so as to perform an ultrasound transmission or reception function. As described above, the first and second transmitting and receiving units 102 and 104 may each perform the function of any one of the ultrasound transmitter (TX) and the ultrasound receiver (RX). The transmission and reception setting unit 706 may set where the functions of the ultrasound transmitter (TX) and the ultrasound receiver (RX) are performed, respectively: the first transmitting and receiving unit 102 or the second transmitting and receiving unit 104. Hereinafter, it is assumed that the first transmitting and receiving unit 102 performs the function of the ultrasound transmitter (TX), and the second transmitting and receiving unit 104 performs the function of the ultrasound receiver (RX).

The distance information managing unit 708 calculates the distance between the first and second transmitting and receiving units 102 and 104 and generates the distance control signal for adjusting the distance between the first and second transmitting and receiving units 102 and 104 according to the calculated distance to transmit the generated distance control signal to the ultrasound or photoacoustic probe 100 or 200. As described above, the object may be, for example, the teeth, the jawbones, or the like. In this case, the ultrasound or photoacoustic probe may have various structures and shapes depending on the kind of subject or object. Therefore, the distance information managing unit 708 may generate the distance control signal for adjusting the distance between the first and second transmitting and receiving units 102 and 104 to transmit the generated distance control signal to the ultrasound or photoacoustic probe 100 or 200. The distance adjusting unit 106 of the ultrasound or photoacoustic probe 100 or 200 may adjust the distance between the first and second transmitting and receiving units 102 and 104 according to the distance control signal received from the distance information managing unit 708.

The distance information managing unit 708 may calculate the distance between the first and second transmitting and receiving units 102 and 104 using a time required for pre-stored information on the medium and the ultrasound transmitted from the ultrasound or photoacoustic probe 100 or 200 to reach the object, and may generate the distance control signal. In general, even though the ultrasound is transmitted having the same frequency from the same position to the object, since a transmission velocity of the ultrasound may be changed depending on the kind of medium, in order to calculate the distance between the first and second transmitting and receiving units 102 and 104, the kind of medium should be considered. Property information of each medium, for example, permittivity, conductivity, density, and the like, of the medium is stored in a database (not illustrated). Particularly, a transmission velocity of the ultrasound of each medium is stored for each frequency of the ultrasound.

The distance information managing unit 708 may control the first transmitting and receiving unit 102 to transmit the ultrasound having a predetermined frequency to the object, in order to calculate the distance between the first and second transmitting and receiving units 102 and 104. The distance information managing unit 708 may acquire the time required for the ultrasound transmitted from the first transmitting and receiving unit 102 to reach the second transmitting and receiving unit 104, and calculate the distance between the first and second transmitting and receiving units 102 and 104 using the transmission velocity of the ultrasound of each medium pre-stored in the database.

Further, the distance information managing unit 708 may calculate the distance between the first and second transmitting and receiving units 102 and 104 from the number of rotations of the above-mentioned micro actuator, and may generate the distance control signal according to the calculated distance. As described above, the distance adjusting unit 106 may include, for example, the micro actuator mounted therein. The distance information managing unit 708 may receive information on the number of rotations of the micro actuator through the above-mentioned sensor, and calculate the distance between the first and second transmitting and receiving units 102 and 104 according to the information on the number of rotations of the micro actuator. The distance information managing unit 708 may calculate the distance between the first and second transmitting and receiving units 102 and 104 from the number of rotations of the micro actuator, and generate the distance control signal to transmit the generated distance control signal to the ultrasound or photoacoustic probe 100 or 200.

In addition, the distance information managing unit 708 may generate information of an ultrasound transmission time difference between one point and another point of the first transmitting and receiving unit 102 to transmit the information to the ultrasound or photoacoustic probe 100 or 200. As described above, the plurality of elements are arrayed in the first transmitting and receiving unit 102, and a distance from each of the elements to the object (a distance of a path through which the ultrasound is transferred to the object) may be different depending on a position of each of the elements. Therefore, there is a need to transmit the ultrasound to the object, with a time difference depending on the position of each of the elements. For example, since an element positioned at an edge of the first transmitting and receiving unit 102 is farther from the object than an element positioned at the center thereof, the element positioned at the edge needs to transmit the ultrasound to the object at a predetermined time earlier than the element positioned at the center. That is, the elements of the first transmitting and receiving unit 102 need to transmit the ultrasound to the object with a predetermined time difference. The distance information managing unit 708 may calculate the distance from each of the element to the object by the above-mentioned method and then, transmit an ultrasound transmission delay time information signal of each of the element to the ultrasound or photoacoustic probe 100 or 200. The first transmitting and receiving unit 102 may transmit the ultrasound from each of the element to the object with a time difference while referring to the ultrasound transmission delay time information.

The signal processing unit 504 controls the signal generating unit 506 to generate the transmission signal for diagnosis of the object or the transmission signal for treatment of the object, and acquires the images of the object using a reception signal received from the ultrasound or photoacoustic probe 100 or 200. As illustrated in FIG. 6, the signal processing unit 504 may include the diagnosis function unit 504-1 and the treatment function unit 504-2. As described above, the ultrasound function controlling unit 704 activates any one of the diagnosis function unit 504-1 and the treatment function unit 504-2 of the signal processing unit 504 according to the input signal inputted in the inputting unit 702. In the case in which the diagnosis function unit 504-1 is activated according to the input signal inputted in the inputting unit 702, the diagnosis function unit 504-1 may control the signal generating unit 506 to generate the transmission signal for diagnosis of the object. Further, in the case in which the treatment function unit 504-2 is activated according to the input signal inputted in the inputting unit 702, the treatment function unit 504-2 may control the signal generating unit 506 to generate the transmission signal for treatment of the object.

In addition, the signal processing unit 504 acquires the images of the object using the reception signal received from the ultrasound or photoacoustic probe 100 or 200. The images may be used, for example, for diagnosis or treatment of the object. The signal transmitting and receiving unit 508 may receive the reception signal, which is the electrical conversion signal of the ultrasound passed through the object, from the ultrasound or photoacoustic probe 100 or 200. The signal transmitting and receiving unit 508 acquires the images of the object in real time using a frequency, a period, intensity, or the like, of the reception signal, and then outputs the images to a display device (not illustrated). The diagnosis function unit 504-1 of the signal processing unit 504 may acquire and output diagnostic images for diagnosis of the object, and the treatment function unit 504-2 of the signal processing unit 504 may acquire and output the images of the object That is, according to the exemplary embodiments of the present invention, the images of the object having a high hardness may be acquired in real time without a risk of exposure to radiation.

The signal generating unit 506 generates the transmission signal, which is the electrical conversion signal of the ultrasound to be transmitted to the object by the ultrasound or photoacoustic probe 100 or 200. As described above, the signal generating unit 506 is controlled by the diagnosis function unit 504-1 or the treatment function unit 504-2 of the signal processing unit 504, and when the diagnosis function unit 504-1 is activated, the signal generating unit 506 generates the transmission signal for diagnosis of the object, and when the treatment function unit 504-2 is activated, the signal generating unit 506 generates the transmission signal for treatment of the object. The transmission signal for diagnosis of the object and the transmission signal for treatment of the object may have, for example, different frequencies from each other. The signal transmitting and receiving unit 508 transmits the transmission signal generated in the signal generating unit 506 to the ultrasound or photoacoustic probe 100 or 200, and receives the reception signal, which is the electrical conversion signal of the ultrasound passed through the object, from the ultrasound or photoacoustic probe 100 or 200.

First, the signal transmitting and receiving unit 508 may transmit the transmission signal generated in the signal generating unit 506 to the ultrasound or photoacoustic probe 100 or 200. In this case, the signal transmitting and receiving unit 508 may transmit the transmission signal to any one of the first and second transmitting and receiving units 102 and 104 of the ultrasound or photoacoustic probe 100 or 200 according to the setting of the transmission and reception setting unit 706. The signal transmitting and receiving unit 508 may transmit the transmission signal to the first or second transmitting and receiving unit 102 or 104 through the interface unit 108 of the ultrasound or photoacoustic probe 100 or 200. The first or second transmitting and receiving unit 102 or 104 of the ultrasound or photoacoustic probe 100 or 200 receiving the transmission signal from the signal transmitting and receiving unit 508 may convert the transmission signal into ultrasound and transmit the ultrasound to the object.

Next, the signal transmitting and receiving unit 508 may receive the reception signal, which is the electrical conversion signal of the ultrasound passed through the object, from the ultrasound or photoacoustic probe 100 or 200. The first or second transmitting and receiving unit 102 or 104 of the ultrasound or photoacoustic probe 100 or 200 may receive the ultrasound passed through the object and convert the ultrasound into the electrical signal, that is, the reception signal. The signal transmitting and receiving unit 508 may receive the reception signal through the interface unit 108 of the ultrasound or photoacoustic probe 100 or 200.

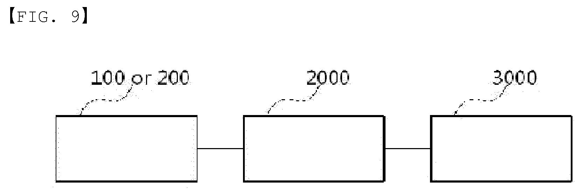

FIG. 9 is a diagram simply illustrating a configuration of an ultrasound system unit 2000 and an external signal input and output apparatus 3000 suitable for the ultrasound or photoacoustic probe according to the present invention. The ultrasound system unit 2000 includes an ultrasound signal or pulse generating unit, an ultrasound signal processing unit, an ultrasound image combining unit, an ultrasound image processing unit, and the like, and the external signal input and output apparatus 3000 includes a keyboard, a printer, a display, a recorder, and the like.

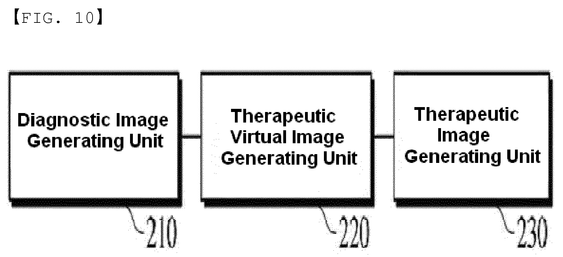

FIG. 10 is a diagram simply illustrating a functional unit generating images using the ultrasound or photoacoustic probe 100 according to the present invention. The image generating unit includes a diagnostic image generating unit 210, a therapeutic virtual image generating unit 220, and a therapeutic image generating unit 230.

The diagnostic image generating unit 210, which is a configuration for acquiring a partial image of the subject or the object in real time, may representatively acquire an ultrasound image, a photoacoustic image, or the like. Here, the acquired image includes a two-dimensional image, a three-dimensional image, a four-dimensional image, or a slice image. In the case in which a diagnostic image generating unit 210 is used for dental purposes, a diagnostic image may be formed using a signal A received in the second face 104 from ultrasound transmitted in the first face 102 and passed through the object. In addition, the diagnostic image may be generated using a signal B received in the first face 102 from ultrasound transmitted from the second face 104 and passed through the object. Further, a synthesized diagnostic image may be formed by combining the received signals A and B. A diagnostic image generating unit 210 transmits the ultrasound signal from the ultrasound or photoacoustic probe to the object and receives the ultrasound signal transmitted through the object, thereby implementing a two-dimensional, three-dimensional, or four-dimensional ultrasound image for the object. The diagnostic image generating unit 210 may segment the real-time diagnostic image into predetermined portions.

The diagnostic image generating unit 210 may receive a signal echoed depending on properties of the medium. When echoed signals of the signals transmitted from the first and second transmitting and receiving units 102 and 104 to the object are defined as an echo-signal a and an echo-signal b, respectively, the diagnostic image generating unit 210 may synthesize the echo-signal a, the echo-signal b, the received signal A, and the received signal B in order to increase sensitivity of the received signal and resolution of the diagnostic image. In this case, as an example of synthesis, a meaningful signal is extracted by overlapping the echo-signal a and the received signal B in a time domain or frequency domain based on a time and a depth. The diagnostic image generating unit 210 serves to generate diagnostic images in real time by variously combining the above-mentioned signals.

The therapeutic virtual image generating unit 220 is a functional unit generating a virtual image of a therapeutic (ultrasound) therapeutic beam field 520 or treatment beam field, or a therapeutic region 510 or treatment beam, and examples of parameters required to visualize the therapeutic (ultrasound) beam include geometry information of the ultrasound or photoacoustic probe 100, a frequency, a focal depth, power intensity, steering, gradient information of the ultrasound or photoacoustic probe 100, the number of channel, a channel group, the number of focus, or the like. A direction and a shape of the therapeutic beam field 520 or treatment beam field and a size, a shape, and energy intensity of the treatment region 510, or the like, may be determined using at least one of the parameters, and a virtual image is formed using the results.

The therapeutic image generating unit 230 may generate a therapeutic image by mapping the diagnostic image generated in the diagnostic image generating unit 210 and the therapeutic virtual image generated in the therapeutic virtual image generating unit 220 according to synchronization information of a system synchronizer 133. In this case, the formed therapeutic image shows where beam and therapeutic region, or treatment beam of the therapeutic virtual image are positioned in real time in a real-time diagnosis state, such that an operator may monitor an operation state in real time while confirming whether or not the therapeutic beam or treatment region is positioned at a desired operation site.

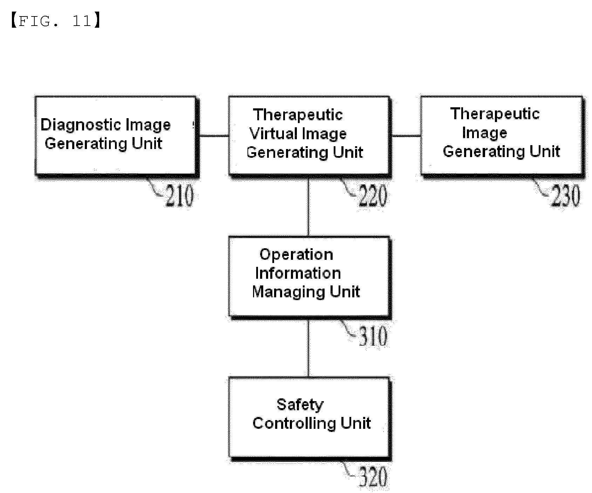

FIG. 11 is a diagram illustrating an example of the image generating unit in which an operation information managing unit 310 and a safety controlling unit 320 are further included.

The operation information managing unit 310 includes information on an operation site, an operation range, and an operation method. The operation method and operation range are determined depending on the operation site. For example, in the case in which the operation site is local, the operation range is narrow, and in the case which the operation site is wide, the operation range is widened. In the case of the treatment pulse transmitted from the first face 102 or the second face 104 is used outside the body such as the teeth or the gum, or used inside the body, the operation method, for example, presence or absence of anesthesia and an anesthesia range, tooth cutting and transmission through the body, or the like, is changed.

In an operation associated with tooth, the operation site of the operation information means the corresponding tooth or nerve to be treated, the operation range means a depth, a length, a width, and the like, of the operation, and the operation method means an operation procedure or operation method for performing endodontic treatment, treatment of periodontal disease, implant, maxillary/mandibular distraction osteogenesis, or the like.

The safety controlling unit 320 serves to prevent an operation tool or region from exceeding the operation range with respect to the operation site, operation range, and operation method set in the operation information managing unit 310. As an example, the safety controlling unit 320 controls a size of the region or allows information to be provided to the operator through a notification signal, so as to transmit the treatment pulse only to the corresponding nerve in the case of treating the nerve. Further, the safety controlling unit provides a risk notification signal or information to the operator through the diagnosis image generated in the diagnostic image generating unit in the case of exceeding the operation range. The notification signal provided at this time may be provided as a sound signal, an image signal, vibration, or the like, so that the operator may sense a risk.

The safety controlling unit 320 may perform a control for preventing the treatment tool or region from deviating from the operation region based on the image generated in the diagnostic image generating unit 210 or the therapeutic image generating unit 230. For example, the safety controlling unit 320 may generate a control signal to control an intensity, a frequency, or an amplitude of the ultrasound transmission signal, the number of transmission channel, a group of the transmission channel, beam-forming conditions (for example, a delay time between channels, or the like), transition, or the like, thereby making it possible to adjust the kind of ultrasound generated in the ultrasound or photoacoustic probe 100, power of the ultrasound, a size of the treatment region, the number of focus, a focal depth, or the like. In this case, the safety controlling unit 320 may use external information or internal information of operation information managing unit 310.

Further, the safety controlling unit 320 may control the intensity, the frequency, the amplitude of the transmission signal, the number of transmission channel, the group of the transmission channel, the beam-forming conditions (for example, the delay time between channels, or the like), transition, or the like, using temperature information in the treatment region, received from a temperature measuring unit 330, thereby making it possible to adjust the kind of ultrasound generated in the ultrasound or photoacoustic probe 100, the power of the ultrasound, the size of the treatment region, the number of focus, the focal depth, or the like.

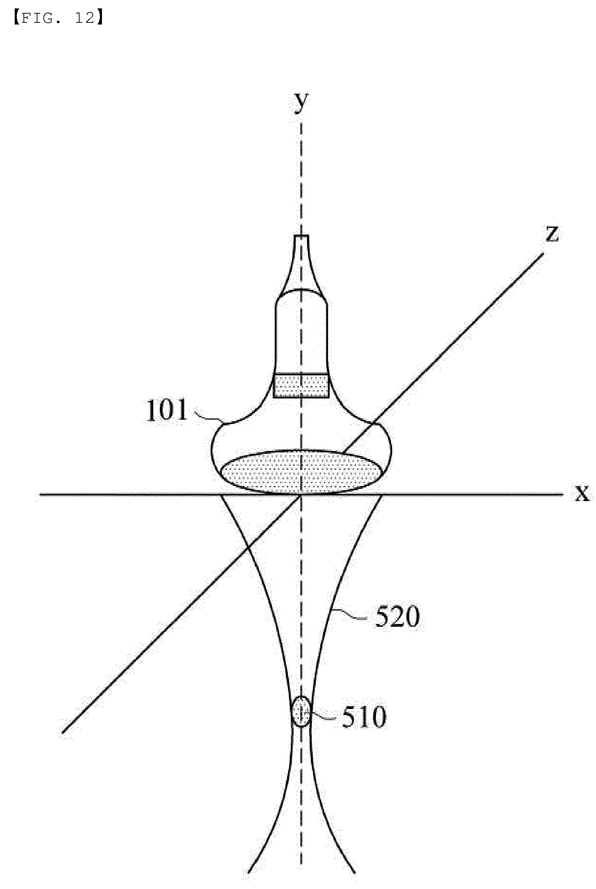

FIG. 12, which illustrates an exemplary embodiment of the present invention, is a diagram virtually illustrating shapes of a therapeutic region 510 or treatment beam, and a therapeutic beam field 520 or treatment beam field, when a convex probe 101 generates an ultrasound signal. The shapes of the therapeutic region 510 or treatment beam, and the therapeutic beam field 520 or treatment beam field may be changed depending on physical geometrical properties and power intensity of the ultrasound or photoacoustic probe, the number of elements, elements groups, physical properties of the element, the focal depth, or the like. The therapeutic region 510 or treatment beam, and the therapeutic beam field 520 or treatment beam field are applied to the object according to values calculated through software simulation (virtual experiment) or a physical experiment. In addition, the therapeutic region 510 or treatment beam, and the therapeutic beam field 520 or treatment beam field are generated in the therapeutic virtual image generating unit 220.

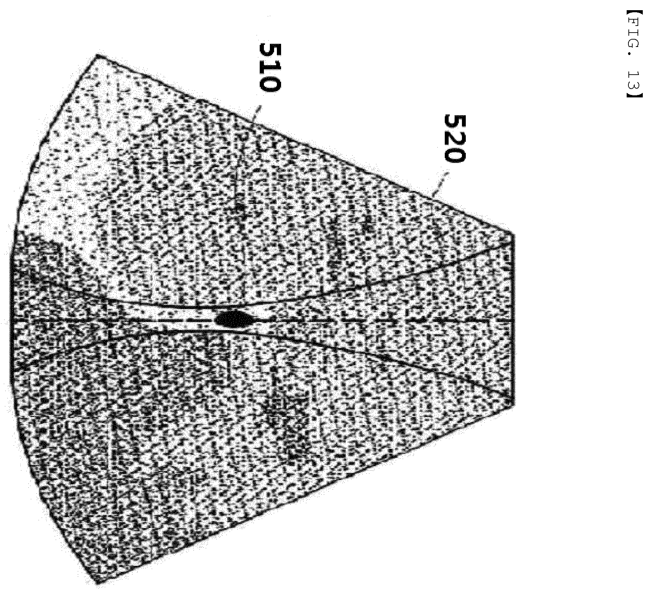

FIG. 13 is a diagram illustrating an image mapped in the therapeutic image generating unit so as to match the image generated in the diagnostic image generating unit 210 and the image generated in the therapeutic virtual image generating unit 220 with real sizes.

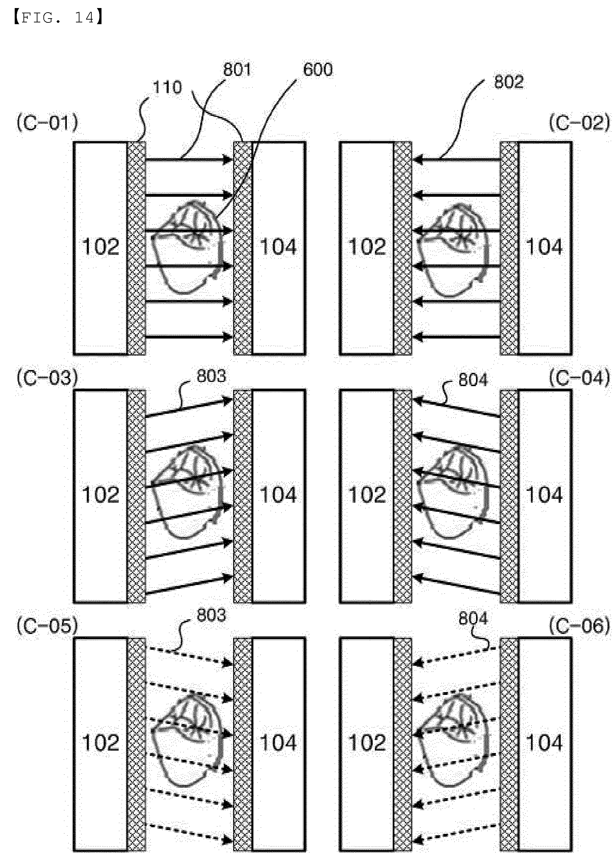

FIG. 14, which illustrates an exemplary embodiment of the present invention, is a diagram illustrating a method of transmitting a signal in any one of the first and second transmitting and receiving units 102 and 104 so as to pass through the object to reach one surface opposite thereto.

(C-01) illustrates a process of a transmission signal 801 generated in the first transmitting and receiving unit 102, transmitted through the object, and received in the second transmitting and receiving unit 104 facing the first transmitting and receiving unit 104, and (C-02) illustrates a process of a transmission signal 802 generated in the second transmitting and receiving unit 104, passed through the object, and received in the first transmitting and receiving unit 102 facing the second transmitting and receiving unit 104.

(C-03) and (C-04) illustrate a state in which directions of the transmission signals are the same as those in (C-01) and (C-02), but right steering is performed, and (C-05) and (C-06) illustrate a state in which left steering is performed.

Although not illustrated, in the case in which the elements of the first and second transmitting and receiving units 102 and 104 are two-dimensionally arranged, steering may be performed upward/downward/left/right. Here, the steering may be controlled by a delay time of sound generated in each of the elements. In this case, image information of the object formed from signals such as 801, 802, 803, 804, 805, 806, and the like, controlled by the delay time illustrated by way of example may reconstruct 2D/3D/4D images.

Particularly, in order to extract the diagnostic image or diagnostic signal from the signals transmitted through the above-mentioned object in the present invention, when there is information on signals transmitted at various angles, effective 3D/4D images may be reconstructed.

Although the exemplary embodiments of the present invention have been disclosed for illustrative purposes, those skilled in the art will appreciate that various modifications, additions and substitutions are possible, without departing from the scope and spirit of the invention as disclosed in the accompanying claims. Accordingly, the scope of the present invention is not construed as being limited to the described embodiments but is defined by the appended claims as well as equivalents thereto.

The present invention may be used to secure the image of the object or to break or treat the object using an ultrasound vibration device. Particularly, the present invention may be used in ultrasound or photoacoustic equipment for securing the image of the object using the signal transmitted through the object to thereby be acquired, in addition to interpreting the signal echoed from the object. As an example, the present invention may be applied to a dental diagnostic imaging system.

### [Detailed Description of Main Elements]

100: ultrasound or photoacoustic probe according to first exemplary embodiment of the present invention
101: example of Convex Probe
102: first transmitting and receiving unit (first face)
104: second transmitting and receiving unit (second face)
106: distance adjusting unit
108: interface unit
110: gel pad
112: protection cover
114: cable
200: ultrasound or photoacoustic probe according to second exemplary embodiment of the present invention
300: image generating unit
210: diagnostic image generating unit
220: therapeutic virtual image generating unit
230: therapeutic image generating unit
310: operation information managing unit
320: safety controlling unit
330: temperature measuring unit
500: ultrasound system
502: setting unit
504: signal processing unit
504-1: diagnosis function unit
504-2: treatment function unit
506: signal generating unit
508: signal transmitting and receiving unit
600: object
702: inputting unit
704: ultrasound function control unit
706: transmission and reception setting unit
708: distance information managing unit
801: transmission signal from first transmitting and receiving unit to second transmitting and receiving unit
802: transmission signal from second transmitting and receiving unit to first transmitting and receiving unit
803: steering transmission signal from first transmitting and receiving unit to second transmitting and receiving unit in right direction
804: steering transmission signal from second transmitting and receiving unit to first transmitting and receiving unit in right direction
805: steering transmission signal from first transmitting and receiving unit to second transmitting and receiving unit in left direction
806: steering transmission signal from second transmitting and receiving unit to first transmitting and receiving unit in left direction
C-01, C-03, C-05: ultrasound transmitted from first transmitting and receiving unit to object, and received in second transmitting and receiving unit
C-02, C-04, C-06: ultrasound transmitted from second transmitting and receiving unit to object, and received in first transmitting and receiving unit

## Claims

1. An ultrasound or photoacoustic probe comprising:
a first transmitting and receiving unit transmitting an ultrasound or photoacoustic signal to an object; and
a second transmitting and receiving unit disposed to face the first transmitting and receiving unit, and receiving the ultrasound or photoacoustic signal passed through the object.

2. The ultrasound or photoacoustic probe of claim 1, further comprising a distance adjusting unit adjusting a distance between the first and second transmitting and receiving units.

3. The ultrasound or photoacoustic probe of claim 1, wherein it has at least one shape of a ' 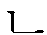 ' shape, a ' 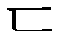 ' shape, a '11' shape, and a '=' shape.

4. The ultrasound or photoacoustic probe of claim 1, wherein each of the first and second transmitting and receiving units includes a plurality of elements generating the ultrasound or photoacoustic signal.

5. The ultrasound or photoacoustic probe of claim 4, wherein the elements generating the ultrasound or photoacoustic signal are elements for diagnosis or treatment.

6. The ultrasound or photoacoustic probe of claim 1, further comprising a wired or wireless transmitting and receiving unit.

7. The ultrasound or photoacoustic probe of claim 1, further comprising a gel pad suitable for properties a medium.

8. The ultrasound or photoacoustic probe of claim 7, wherein the gel pad has a shape corresponding to the ultrasound or photoacoustic probe.

9. An ultrasound diagnosis system using the ultrasound or photoacoustic probe of any one of claims 1 to 8, the ultrasound diagnosis system comprising:
a diagnosis function unit;
a signal generating unit generating a diagnosis pulse for diagnosis of the object when the diagnosis function unit is activated; and
a diagnostic image generating unit generating a diagnostic image for diagnosis of the object using the diagnosis pulse.

10. An ultrasound therapy system using the ultrasound or photoacoustic probe of any one of claims 1 to 8, the ultrasound therapy system comprising:
a treatment function unit; and
a signal generating unit generating a treatment pulse for treatment of the object when the treatment function unit is activated.

11. An ultrasound diagnosis and therapy system using the ultrasound or photoacoustic probe of any one of claims 1 to 8, the ultrasound diagnosis and therapy system comprising:
a diagnosis function unit;
a treatment function unit;
a signal generating unit generating a diagnosis pulse for diagnosis of the object when the diagnosis function unit is activated, and generating a treatment pulse for treatment of the object when the treatment function unit is activated;
a diagnostic image generating unit generating a diagnostic image for diagnosis of the object using the diagnosis pulse;
a therapeutic virtual image generating unit generating a therapeutic virtual image for treatment of the object using the treatment pulse; and
a therapeutic image generating unit generating a therapeutic image using the diagnostic image and the therapeutic virtual image.

12. An ultrasound or photoacoustic system using the ultrasound or photoacoustic probe of any one of claims 1 to 8, the ultrasound or photoacoustic system comprising a therapeutic virtual image generating unit generating virtual images of a therapeutic region or treatment beam, or a therapeutic beam field or treatment beam field, of a transmission signal for treatment.

13. The ultrasound or photoacoustic system of claim 12, further comprising an operation information managing unit including information on at least one of an operation site, an operation range, and an operation method.

14. The ultrasound or photoacoustic system of claim 13, further comprising a safety controlling unit securing safety of an operation according to the information of the operation information managing unit.

15. The ultrasound or photoacoustic system of claim 12, further comprising:
a diagnosis function unit;
a treatment function unit; and
a signal generating unit generating a diagnosis pulse for diagnosis of the object when the diagnosis function unit is activated, and generating a treatment pulse for treatment of the object when the treatment function unit is activated,
wherein a signal generated in the signal generating unit has a delayed signal value so that any one of the first and second transmitting and receiving units has directivity and generates a steering signal, and a 2D/3D/4D image is formed by analyzing the received signal according to the delayed signal value.

16. The ultrasound or photoacoustic system of claim 15, wherein the signal generated in the signal generating unit is controlled so as to be steered to one surface disposed to face any one of the first and second transmitting and receiving units in any one direction of upward, downward, left, and right directions.
